# EUROPEAN PATENT APPLICATION

(11) **EP 4 273 311 A1**
(43) Date of publication of application: **08.11.2023**
(21) Application number: 21914748.5
(22) Date of filing: 22.12.2021
(51) Int. Cl.: D03D 13/00, D03D 15/283, D03D 15/43, D03D 15/56, D03D 17/00, A61L 15/42, A61F 13/00

(54) **COMPRESSION GARMENT**

(30) Priority: 29.12.2020 ES 202032808 U
(71) Applicant: Asociación de Investigación de la Industria Textil (AITEX), 03801 Alcoy (Alicante) (ES)
(72) Inventor: BAEZA GARCÍA, Ramón, 03801 Alcoy (Alicante) (ES); FAGES SANTANA, Eduardo, 03801 Alcoy (Alicante) (ES); CAMBRA SÁNCHEZ, Vicente, 03801 Alcoy (Alicante) (ES); BLANES MARTÍNEZ, María del Mar, 03801 Alcoy (Alicante) (ES); GISBERT RUIZ, María José, 03801 Alcoy (Alicante) (ES); BLANES COMPANY, María, 03801 Alcoy (Alicante) (ES)
(74) Representative: Ibarra Garcia, Isabel
(86) International application number: PCT/ES2021/070927
(87) International publication number: WO 2022/144478

(57) **Abstract**

The present invention discloses a compression garment with therapeutic properties made up of a double layer jersey-based weft-knitted fabric with a structure formed by three courses. The first course is a mesh of multifilament polyamide yarns, made up of a weft yarn longitudinally without weaving, the second course is made by means of the weft yarn inserted longitudinally without weaving and secured between the multifilament polyamide yarns forming the mesh, wherein the weft yarn is an elastic yarn with an elastane core and an outer polyamide coating with a thickness of 600 dtex, and the third course is an elastic yarn with an elastane core and an outer polyamide coating with a thickness of 200 dtex. Advantageously, using a yarn with reduced thickness in the third course with respect to the second course provides breathability and thermal comfort.

## Description

### OBJECT OF THE INVENTION

The present invention consists of a compression garment with therapeutic properties intended preferably for the treatment of lipedema disease. Specifically, the garment of the invention is made up of a weft-knitted fabric in which a weft yarn is longitudinally inserted without weaving, with the longitudinal axis of the garment being located parallel to the warp direction of the fabric, which allows exerting the containment and compression required in the patient's limbs without causing the constriction thereof or the twisting of the fabric.

Advantageously, the proposed garment is inexpensive and effective by allowing the standardization thereof by sizes and offering an aesthetic external appearance allowing it to be worn as the only garment.

### BACKGROUND OF THE INVENTION

Compression garments are frequently used today as treatment indicated for the therapy of lipedema and other circulatory or fatty tissue diseases.

Lipedema is a progressive fatty tissue disease characterized by pathological fat accumulation, predominantly in the arms and legs. Containment treatments by means of compression garments have proven to be effective in the treatment of this pathology. The level of pressure achieved with these garments, i.e., the compression exerted by these garments on the patient's or user's limbs, depends on several factors such as the physical-mechanical properties of the material of the garments (fabric structure, yarns used, etc.) and the morphology and volumetry of the patient's limb, as well as other properties such as the hardness thereof.

In that sense, the level of compression is directly proportional to the stress applied with the garment and inversely proportional to the size of the limb on which the garment is worn.

The production of the fabric used in compression garments of this type allows them to be classified as circular-knitted and flat-knitted garments, the latter also being known as weft-knitted garments.

Circular fabric is characterized by not having any seam, being thinner and more aesthetic garments. The drawback of compression garments made of circular fabric is that they are prone to exert a tourniquet effect or rotational stress, wrapping around the limb and constricting it.

Likewise, compression garments made of circular knit use a spiral weft, the weft yarns of which are arranged in different horizontal planes. When a strain is applied on said yarns, they tend to stretch and therefore the pitch and diameter of the spiral are modified to compress the limb. In fact, this generates a form of compression that is difficult to control, causing what is known as the tourniquet effect, which means that it is not exerting the same pressure on the entire limb, a notable drawback that confirms the recommendation to use flat-knitted or weft-knitted garments instead of circular knit.

However, known flat-knitted compression garments are expensive because they must be custom-made. Additionally, both known circular-knitted and weft-knitted garments have an unaesthetic finish, which makes it necessary to wear an additional garment to cover them, therefore making them uncomfortable and causing the user to experience excessive heat.

Based on the foregoing, the applicant of the present utility model has found the need to develop a compression garment formed by a weft-knitted or flat-knitted fabric with structural and aesthetic characteristics such that they present a certain adaptation to different patients.

### DESCRIPTION OF THE INVENTION

The compression garment of the invention is preferably formed by way of a pair of pants or the like, however, the present invention is in no case limited to garments of this type.

In order to provide a compression garment with the therapeutic properties required for the treatment of lipedema, the garment of the invention is made with a fabric formed by three courses of a double layer jersey-based weft-knitted structure, also known as flat-knitted double layer jersey-based structure, to which a weft yarn is integrated longitudinally, i.e., without weaving.

As mentioned, the structure is obtained in double layer jersey-based knitting equipment in which each needle bed has a plurality of needles and plates to perform the knitting operation.

The first course of the structure forming the fabric is a mesh preferably made with multifilament polyamide yarns, and provides the body of the compression garment, since this course represents the highest percentage in the fabric forming the compression garment.

The second course is made by means of a weft yarn longitudinally integrated in the mesh, i.e., without weaving, being secured between the multifilament polyamide yarns forming the mesh of the first course. This weft yarn is preferably an elastic yarn with an elastane core and an outer polyamide coating with a thickness of at least 600 dtex (decitex). This weft yarn provides elasticity to the final fabric, and particularly recovery capacity, this being the property which allows offering the compression required in the patient's limb on which it is applied.

Finally, the third course of the structure is made by means of an elastic yarn, preferably an elastic yarn with an elastane core and an outer polyamide coating with a thickness of at least 200 dtex. This yarn of the third course provides elasticity and recovery in the weft direction and in the warp direction of the fabric. Advantageously, using a yarn with reduced thickness in the third course with respect to the second course reduces the thickness of the fabric with respect to other known fabrics, providing breathability and thermal comfort thereto.

A compression garment, preferably by way of a pair of pants or the like, such as leggings, is made with the fabric described above. However, the present invention is in no case limited to garments of this type and can be alternatively configured as a garment for the upper limbs and/or torso of the user.

Advantageously, in the compression garment of the present invention the yarns of the weft are contained in the horizontal planes, and therefore upon exerting a force in said direction, the yarns stretch uniformly in said direction. This configuration of the compression garment prevents the twisting and constriction of the legs and ensures the absence of the tourniquet effect.

Moreover, the longitudinal axis of the compression garment by way of a pair of pants is located parallel to the warp direction of the fabric. This means that the elasticity of the garment is greater in the direction transverse to the warp, and therefore compression is greater in this direction.

Another aspect to be highlighted with respect to the present invention is that the compression garment is made up of a minimal number of parts, preferably two parts in the garment configured by way of a pair of pants, so as to minimize the number of seams, which results in greater user comfort. In the case of the garment configured by way of a pair of pants, the two parts form the legs of the compression garment and are joined by only two seams arranged parallel to the longitudinal axis of the garment, with one seam running along the inner face of both legs, whereas the second seam runs along the front and back rises of the compression garment.

Optionally, and in order to eliminate bulges caused by the seams and result in greater user comfort, the seams of the compression garment have a riveted finish on the inner surface of the garment, whereas a seam-binding tape is arranged over the seams on the outer surface thereof, giving rise to flat seams.

The proposed garment offers the possibility of being manufactured by standardized sizes, which makes it more economical for the end user compared to conventional custom-made garments. Moreover, it should be pointed out that the compression garment is optionally provided with an adjustable waist in order to adapt to the largest number of users possible and their different morphologies.

As described in detail above, the structure of the fabric of the compression garment and the production thereof allow wearing the garment directly as it offers an improved aesthetic appearance, preventing the need to use a second garment over the compression garment, which translates into greater convenience and thermal comfort for the user. In that sense, the compression garment optionally includes decorative elements which increase the aesthetic value thereof and which are fixed to the fabric by means of suitable techniques that do not change the elasticity of the fabric or its therapeutic properties.

Finally, it should be pointed out that the compression garment of the present invention is more durable as it is made up of yarns with considerable thickness.

### DESCRIPTION OF THE DRAWINGS

To complement the description that will be made below and for the purpose of helping to better understand the features of the invention according to a preferred practical embodiment thereof, there is attached as an integral part of said description a set of drawings in which the following is depicted in an illustrative and non-limiting manner:
Figure 1 shows a front view of a compression garment according to a preferred embodiment of the present invention, in which the compression garment is configured by way of a pair of pants.
Figure 2 shows the exploded view of the compression garment illustrated in Figure 1, in which the detail of the lines forming the seams can be seen.
Figure 3 shows a view of the structure of the fabric forming the compression garment according to a preferred embodiment of the invention.

### PREFERRED EMBODIMENT OF THE INVENTION

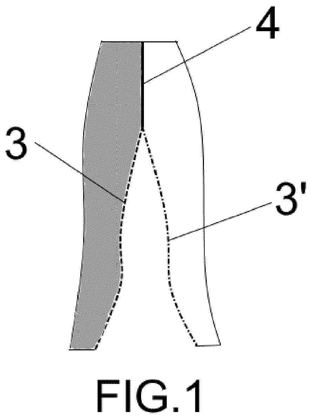

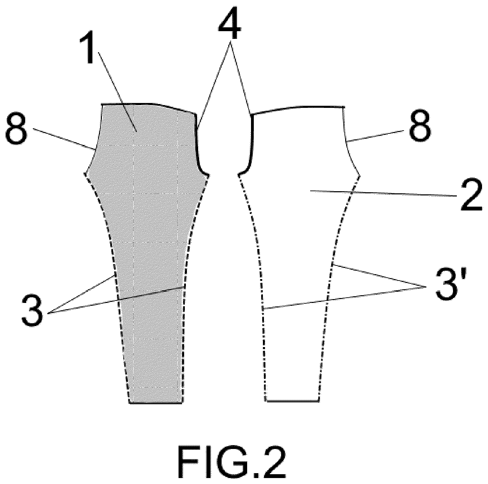

Figures 1 and 2 shown an embodiment of the invention in which the compression garment is configured by way of a pair of pants from two parts (1), (2).

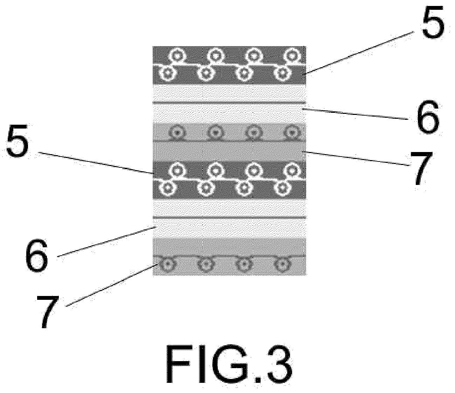

The fabric of the parts is a double layer jersey-based weft-knitted fabric with a structure formed by three courses. The different courses forming the structure can be seen in the preferred embodiment of the invention illustrated in Figure 3. The first course (5) is a mesh of multifilament polyamide yarns providing the body of the garment and in which a weft yarn is integrated longitudinally without weaving. In this first course (5), the mesh has a configuration commonly known in the textile sector as an 'RR' structure.

The second course (6) is made by means of a weft yarn inserted longitudinally in the mesh forming the structure of the fabric. This weft yarn is an elastic yarn with an elastane core and an outer polyamide coating with a thickness of 600 dtex. As described in detail above, the weft yarn is not woven, i.e., secured between the multifilament polyamide yarns forming the mesh of the first course (5). According to the present embodiment of the invention, the elastane content of the elastic weft yarn is between 18% and 21%.

Finally, the third course (7) of the structure is made by means of an elastic yarn, preferably an elastic yarn with an elastane core and an outer polyamide coating with a thickness of at least 200 dtex. In the preferred embodiment illustrated in Figure 3, it can be seen how the structure forming the fabric is formed with two cycles of the three courses, since the third course (7) is applied on one of the faces of the fabric with one of the needle beds in the first cycle, whereas in the second cycle the third course (7) is applied to the other face of the fabric.

Moreover, as observed in the embodiment of the invention depicted in Figures 1 and 2, the compression garment by way of a pair of pants is made up of two parts (1), (2), which form the legs of the garment and are joined by only two seams arranged parallel to the longitudinal axis of the garment, with one seam running along the inner face of both legs, whereas the second seam runs along the front and back rises of the compression garment. In that sense, Figure 2 shows the parts (1), (2) before they are sewn to make the garment. One of the two seams of the garment runs along the inner face of both legs, joining the ends (3) of the part (1) and the ends (3') of the other part (2). The second seam runs along the front and back rises of the compression garment, joining the ends (4) of the front rise and the ends (8) of the back rise of the garment by way of a pair of pants.

Preferably, these seams intersect in the area of the user's pelvis, therefore causing them to coincide with a hard area of the body to result in greater user comfort.

The configuration of the fabric and the composition of the yarns forming same, together with the design of the compression garment described according to the present preferred embodiment of the invention, provide a garment in which pressure of between 23 and 32 mm Hg is applied in the portion coinciding with the ankles. However, the present invention is in no case limited to this pressure range, since other pressure ranges required based on the degree by which the patient is affected could be achieved by varying the composition of the elastic weft yarns.

According to an embodiment of the invention, the compression garment is high waisted, i.e., with a waist arranged above the patient's hips. Additionally, the waist of the compression garment is provided with an adjustable waist made up of an elastic band with eyelets.

The aesthetic value of the compression garment object of the present invention is an important factor as it allows wearing the garment directly, without having to use a second garment that covers the compression garment, with the subsequent discomfort and high temperature experienced by the user. It is for this reason that, according to an embodiment of the invention, the compression garment includes decorative elements, such as beading, vinyl materials, or straps, which are fixed to the compression garment taking into consideration the nature thereof.

In this way, in an embodiment of the invention, the compression garment includes decorative elements fixed by means of heat treatment known as heat setting, using a temperature that is lower than the limit temperature which modifies the elasticity of the fabric permanently.

Alternatively, in another embodiment of the invention, the decorative elements can be added to the garment by means of sewing. To that end, a zigzag stitch is used since it allows the stretching the garment without causing the yarn to break. Another embodiment of the invention contemplates sewing the decorative elements by means of a strong elastic yarn using flat stitches, which prevents the appearance of bulges in the garment.

Ultimately, the compression garment of the present invention provides a long-lasting, strong, and functional therapeutic solution with a high aesthetic value which allows applying containment therapy on patients affected by lipedema, all this with a low economic cost with respect to other known solutions.

## Claims

1. A compression garment, **characterized in that** it is made with a fabric formed by at least three courses of a double layer jersey-based weft-knitted structure to which a weft yarn has been integrated longitudinally without weaving, with the longitudinal axis of the garment being located parallel to the warp direction of the fabric; wherein the first course (5) is a mesh made by means of multifilament polyamide yarns to obtain the body of the compression garment; the second course (6) is made by means of the weft yarn inserted longitudinally without weaving and secured between the multifilament polyamide yarns forming the mesh, wherein the weft yarn is an elastic yarn with an elastane core and an outer polyamide coating with a thickness of at least 600 dtex; and the third course (7) is made by means of an elastic yarn with an elastane core and an outer polyamide coating with a thickness of at least 200 dtex.

2. The compression garment according to claim 1, **characterized in that** the weft yarn is an elastic yarn with an elastane core, wherein the elastane core is present in a percentage of between 18% and 21%.

3. The compression garment according to claim 1, **characterized in that** it is formed by way of a pair of pants or the like from two parts (1), (2) which form the legs of the compression garment, such that the parts (1), (2) are joined by seams arranged parallel to the longitudinal axis of the compression garment, wherein only two seams join the two parts, with one seam running along the inner face of both legs and a second seam running along the front and back rises of the compression garment.

4. The compression garment according to claim 3, **characterized in that** the seams have a riveted finish on the inner surface of the compression garment and a seam-binding tape arranged on the seams at the outer portion of the garment.

5. The compression garment according to claim 3, **characterized in that** it is provided with an adjustable waist.

6. The compression garment according to claim 5, **characterized in that** the adjustable waist is made up of an elastic band with eyelets.

7. The compression garment according to claim 3, **characterized in that** it is high waisted.

8. The compression garment according to claim 1, **characterized in that** it includes decorative elements fixed by means of heat at a temperature below the limit temperature which modifies the elasticity of the fabric permanently.

9. The compression garment according to claim 1, **characterized in that** it includes decorative elements sewn using a zigzag stitch.

10. The compression garment according to claim 1, **characterized in that** it includes decorative elements sewn using a flat stitch with strong elastic yarn.

11. The compression garment according to claim 3, **characterized in that** it applies a pressure of between 23 and 32 mmHg in the portion coinciding with the ankles.
